# EUROPEAN PATENT SPECIFICATION

(11) **EP 1 963 858 B1**
(45) Date of publication and mention of the grant of the patent: **28.09.2016**
(21) Application number: 06831482.2
(22) Date of filing: 22.12.2006
(51) Int. Cl.: G01N 33/569

(54) **A BIOASSAY AND PEPTIDES FOR USE THEREIN**
BIOASSAY UND PEPTIDE ZUR VERWENDUNG DARIN
DOSAGE BIOLOGIQUE ET PEPTIDES UTILISES DANS CELUI-CI

(30) Priority: 23.12.2005 GB 0526273
(43) Date of publication of application: 03.09.2008
(73) Proprietor: Rapid Biosensor Systems Ltd, Cambridge CB2 4AT (GB)
(72) Inventor: SHARMA, Ram, P., Comberton, Cambridge CB3 7DS (GB); MEHROTRA, Amit, P., Comberton, Cambridge CB3 7DS (GB)
(74) Representative: Norris, Timothy Sweyn
(86) International application number: PCT/GB2006/004931
(87) International publication number: WO 2007/072063

(56) References cited:
- EP-A- 1 491 553
- WO-A-00/73345
- WO-A-03/038444
- WO-A-2004/108948
- US-A- 6 033 904
- US-A1- 2002 131 975
- US-B1- 6 245 331
- PAO YA-LAN ET AL: "Effect of serine O-glycosylation on cis-trans proline isomerization" BIOCHEMICAL AND BIOPHYSICAL RESEARCH COMMUNICATIONS, vol. 219, no. 1, 1996, pages 157-162, XP002458770 ISSN: 0006-291X

### SEQUENCE LISTING

<110> Rapid Biosensor Systems Ltd.
<120> Assay System
<130> A483P021
<160> 26
<170> PatentIn version 3.3
<210> 1
   <211> 9
   <212> PRT
   <213> Mycobacterium tuberculosis
<400> 1
   Gly Arg Asp Ile Lys Val Gln Phe Gln
   1 5
<210> 2
   <211> 9
   <212> PRT
   <213> Mycobacterium tuberculosis
<400> 2
   Arg Asp Ile Lys Val Gln Phe Gln Ser
   1 5
<210> 3
   <211> 9
   <212> PRT
   <213> Mycobacterium tuberculosis
<400> 3
   Asp Ile Lys Val Gln Phe Gln Ser Gly
   1 5
<210> 4
   <211> 9
   <212> PRT
   <213> Mycobacterium tuberculosis
<400> 4
   Ile Lys Val Gln Phe Gln Ser Gly Gly
   1 5
<210> 5
   <211> 9
   <212> PRT
   <213> Mycobacterium tuberculosis
<400> 5
   Lys Val Gln Phe Gln Ser Gly Gly Asn
   1 5
<210> 6
   <211> 9
   <212> PRT
   <213> Mycobacterium tuberculosis
<400> 6
   Val Gln Phe Gln Ser Gly Gly Asn Asn
   1 5
<210> 7
   <211> 9
   <212> PRT
   <213> Mycobacterium tuberculosis
<400> 7
   Gln Phe Gln Ser Gly Gly Asn Asn Ser
   1 5
<210> 8
   <211> 9
   <212> PRT
   <213> Mycobacterium tuberculosis
<400> 8
   Phe Gln Ser Gly Gly Asn Asn Ser Pro
   1 5
<210> 9
   <211> 9
   <212> PRT
   <213> Mycobacterium tuberculosis
<400> 9
   Gln Ser Gly Gly Asn Asn Ser Pro Ala
   1 5
<210> 10
   <211> 9
   <212> PRT
   <213> Mycobacterium tuberculosis
<400> 10
   Ser Gly Gly Asn Asn Ser Pro Ala Val
   1 5
<210> 11
   <211> 18
   <212> PRT
   <213> Mycobacterium tuberculosis
<400> 11
   Gly Arg Asp Ile Lys Val Gln Phe Gln Ser Gly Gly Asn Asn Ser Pro
   1 5 10 15
   Ala Val
<210> 12
   <211> 18
   <212> PRT
   <213> Mycobacterium tuberculosis
<400> 12
   Tyr Leu Gln Val Pro Ser Pro Ser Met Gly Arg Asp Ile Lys Val Gln
   1 5 10 15
   Phe Gln
<210> 13
   <211> 6
   <212> PRT
   <213> Mycobacterium tuberculosis
<400> 13
   Gly Asn Asn Ser Pro Ala
   1 5
<210> 14
   <211> 6
   <212> PRT
   <213> Mycobacterium tuberculosis
<400> 14
   Asn Asn Ser Pro Ala Val
   1 5
<210> 15
   <211> 9
   <212> PRT
   <213> Artificial Sequence
<220>
   <223> Modification (at position 9) of native Mycobacterium tuberculosis
   sequence
<400> 15
   Ser Gly Gly Asn Asn Ser Pro Ala Ala
   1 5
<210> 16
   <211> 9
   <212>PRT
   <213> Artificial Sequence
<220>
   <223> Modification (at position 9) of native Mycobacterium tuberculosis
   sequence
<400> 16
   Ser Gly Gly Asn Asn Ser Pro Ala Phe
   1 5
<210> 17
   <211> 9
   <212> PRT
   <213> Artificial Sequence
<220>
   <223> Modification (at position 9) of native Mycobacterium tuberculosis
   sequence
<400> 17
   Ser Gly Gly Asn Asn Ser Pro Ala Leu
   1 5
<210> 18
   <211> 9
   <212> PRT
   <213> Artificial Sequence
<220>
   <223> Modification (at position 9) of native Mycobacterium tuberculosis
   sequence
<400> 18
   Ser Gly Gly Asn Asn Ser Pro Ala Met
   1 5
<210> 19
   <211> 9
   <212> PRT
   <213> Artificial Sequence
<220>
   <223> Modification (at position 9) of native Mycobacterium tuberculosis
   sequence
<400> 19
   Ser Gly Gly Asn Asn Ser Pro Ala Tyr
   1 5
<210> 20
   <211> 9
   <212> PRT
   <213> Artificial Sequence
<220>
   <223> Modification (at position 9) of native Mycobacterium tuberculosis sequence
<400> 20
   Ser Gly Gly Asn Asn Ser Pro Ala Gly
   1 5
<210> 21
   <211> 9
   <212> PRT
   <213> Artificial Sequence
<220>
   <223> Modification (at position 9) of native Mycobacterium tuberculosis sequence
<400> 21
   Ser Gly Gly Asn Asn Ser Pro Ala Ser
   1 5
<210> 22
   <211> 9
   <212> PRT
   <213> Artificial Sequence
<220>
   <223> Modification (at position 9) of native Mycobacterium tuberculosis sequence
<400> 22
   Ser Gly Gly Asn Asn Ser Pro Ala Pro
   1 5
<210> 23
   <211> 9
   <212> PRT
   <213> Artificial Sequence
<220>
   <223> Modification (at position 9) of native Mycobacterium tuberculosis
   sequence
<400> 23
   Ser Gly Gly Asn Asn Ser Pro Ala Asn
   1 5
<210> 24
   <211> 9
   <212> PRT
   <213> Artificial Sequence
<220>
   <223> Modification (at position 9) of native Mycobacterium tuberculosis
   sequence
<400> 24
   Ser Gly Gly Asn Asn Ser Pro Ala Glu
   1 5
<210> 25
   <211> 9
   <212> PRT
   <213> Artificial Sequence
<220>
   <223> Modification (at position 9) of native Mycobacterium tuberculosis
   sequence
<400> 25
   Ser Gly Gly Asn Asn Ser Pro Ala Arg
   1 5
<210> 26
   <211> 9
   <212> PRT
   <213> Artificial Sequence
<220>
   <223> Modification (at position 9) of Mycobacterium tuberculosis
   sequence
<220>
   <221> misc_feature
   <222> (9)..(9)
   <223> Xaa can be any naturally occurring amino acid
<400> 26
   Ser Gly Gly Asn Asn Ser Pro Ala Xaa
   1 5

## Description

### Field of the Invention

The invention relates to peptides for use in an assay for the detection of Mycobacterium tuberculosis, and also to the assay itself.

### Background and Prior Art known to the Applicant

Tuberculosis is an infection caused by the bacterium *Mycobacterium tuberculosis.* Tuberculosis is a major problem in many countries, especially in the developing world, and is on the increase in many developed countries. Whilst tuberculosis often presents as a lung infection, it can also affect other parts of the body such as lymph nodes, skin and bones.

Diagnosis of tuberculosis, so allowing treatment, is the major weapon against the spread of the disease. Diagnosis may be made using a combination of clinical signs, sputum cultures, chest X-ray, histology of tissue and bronchial lavage fluid, and the use of the tuberculin skin test (also known as the Purified Protein Derivative Standard, or PPD skin test) such as the Mantoux test and Heaf Test. The tests rely on an immune reaction to M. tuberculosis protein, revealing the presence of antibodies of *M. tuberculosis* in the patient's blood.

In an example, the US20020131975 publication relates to a vaccinating agent for use in promoting an effective immune response, in a mammalian host, against an infectious pathogen from the genus Mycobacterium. The vaccinating agent includes at least one immunodominant epitope of at least one majorly abundant extracellular product selected from the group consisting of *M. Tuberculosis* 110 KD protein, 80 KD protein, 71 KD protein, 58 KD protein, 45 KD protein, 32A KD protein, 32B KD protein, 30 KD protein, 24 KD protein, 23.5 KD protein, 23 KD protein, 16 KD protein, 14 KD protein, 12 KD protein and respective analogs, homologs, and subunits thereof.

The tests require administration of an immunological challenge to a patient, and a subsequent follow-up examination to determine the test result. These steps make the test difficult to apply for mass screening operations.

The present invention attempts to provide a solution to some of these problems.

### Summary of the Invention

In an aspect, embodiments of the present disclosure provide a peptide consisting of any of the following sequences:
(SEQ ID 15)
   SGGNNSPAA;
(SEQ ID 17)
   SGGNNSPAL;
(SEQ ID 18)
   SGGNNSPAM.

Optionally, the peptide further comprising a label.

In another aspect, embodiments of the present disclosure provide an antibody that specifically binds to SEQ ID 11, or a fragment of said antibody, wherein said fragment specifically binds to SEQ ID 11.

In a broad first aspect, the invention comprises a peptide of fewer than 18 amino acids comprising the sequence NSPAX where X is Methionine (SEQ ID 18), Leucine (SEQ ID 17), Alanine (SEQ ID 15) or Valine (SEQ ID 10) wherein the peptide is capable of binding to an antibody raised against the peptide GRDIKVQFQSGGNNSPAV (SEQ ID 11). In particularly preferred embodiments, the peptide binds to such an antibody.

In practical terms, such an antibody would be raised against this peptide (SEQ ID 11) supplemented by an additional C amino acid at the C-terminal end, i.e. raised against GRDIKVQFQSGGNNSPAVC.

In a second aspect, therefore, the peptide comprises up to 6 additional amino acids at the C and/or N terminal end of the sequence NSPAX.

Preferably, therefore, the peptide of the first or second aspect comprises the sequence NNSPAX, and more preferably the peptide comprises the sequence NNSPAV (SEQ ID 14).

In a further preferred aspect, the peptide comprises the sequence SGGNNSPAX (SEQ ID 26), where X is Methionine (SEQ ID 18), Leucine (SEQ ID 17), Alanine (SEQ ID 15) or Valine (SEQ ID 10).

In any aspect of the invention it is preferred that the peptide is not GRDIKVQFQSGGNNSPAV (SEQ ID 11).

Also in any aspect of the invention it is preferable that the peptide consists of the sequence SGGNNSPAX (SEQ ID 26), where X is Methionine (SEQ ID 18), Leucine (SEQ ID 17), Alanine (SEQ ID 15) or Valine (SEQ ID 10).

Also in any peptide of the invention it is preferable that X is Methionine (SEQ ID 18), Leucine (SEQ ID 17) or Alanine (SEQ ID 15). Especially preferred are peptides wherein X is Methionine (SEQ ID 18) or Alanine (SEQ ID 15). These particular substitutions are found to have particularly good Leu-heat stability, making them suitable for field use in hot countries.

Particularly preferred peptides according to any aspect of the invention further comprise a label. The use of a labelled peptide facilitates its use in an assay for the detection of *Mycobacterium tuberculosis.* Suitable labels might include radio-labels (e.g. by the addition of a radioactive species or the substitution of one or more atoms of the peptide by a its radioactive equivalent) thereby allowing the presence or concentration of the peptide, or a complex comprising the peptide, to be readily detected, e.g. by scintillation counting or the like. Other labels such as an enzyme-mediated chromogenic label might also be used. Particularly preferred labels would be fluorescent labels, including binding of the peptide to a fluorescent protein. A fluorescent label such as Alexa Fluor (e.g. Alexa Fluor 633) is, however, particularly suitable.

Preferably also, any of the above peptides are in an isolated form, i.e. essentially free of proteins of native (e.g. bacterial, fungal or mammalian) origin.

Peptides described above have application in assays for the detection of *Mycobacterium.*

Included within the scope of the invention is a nucleotide sequence encoding any of the above peptides. Such a sequence has application in the biosynthesis of the peptides of the present invention.

Included within the scope of the invention is an antibody raised against SEQ ID 11. The antibodies of the invention may be polyclonal or monoclonal, or may be recombinant antibodies, such as chimeric antibodies wherein the murine constant regions on light and heavy chains are replaced by human sequences, or CDR-grafted antibodies wherein only the complementary determining regions are e.g. of murine origin. Antibodies of the invention may also be human antibodies prepared, for example, by immunization of transgenic animals capable of producing human antibodies (see, for example, PCT Application No. WO93/12227).

Also included within the scope of the invention is a displacement ELISA (Enzyme- Linked Immuno-Sorbent) assay for determining the presence of *Mycobacteriurn tuberculosis* comprising a peptide according to any preceding claim. A suitable procedure for carrying out such an assay will be described below. Preferably, such a displacement assay further comprises an antibody raised against SEQ ID 11, or a fragment of such an antibody having affinity for SEG ID 11.

Also included within the scope of the invention is an ELIZA assay for determining the presence of *Mycobacterium tuberculosis* comprising an antibody raised against SEQ ID 11, or a fragment of such an antibody having affinity for SEG ID 11.

Further included within the scope of the invention is a displacement assay for determining the presence of *Mycobacterium tuberculosis* comprising a peptide according to the invention and a peptide according to SEQ ID 11.

### Test Development and Description of Preferred Embodiments

The test is based upon the identified T-cell epitope from *M. tuberculosis* Ag85B [Mustafa, A.S. et al, "Identification and HL Restriction of Nuturally Derived ThI -Cell Epitopes from the Secreted 1-Cell Epitopes from the Secreted Mycobacterium tuberculosis Antigen 85B Recognised by Antigen-Specific Human CD4+ T-Cell Lines", Infection and Immunity, 68(7), 3933-3940, 2000] represented by the 18-mer peptide p3 (Amino acids 19 to 36, denoted P779) with the sequence GRDIKVQFQSGGNNSPAV (SEQ ID 11).

An aspect of the invention is the provision of a competitive displacement ELISA (enzyme-linked immunosorbent assay) assay - effectively a ligand displacement assay. Antibodies to *M. tuberculosis,* particularly those directed towards the Ag85B epitope, are bound to a test plate. Peptides capable of binding to the antibodies, but with a lower affinity than the *M. tuberculosis* antigen are bound to the antibodies, preferably labelled with e.g. a fluorescent marker. When samples containing M. tuberculosis antigens (e.g. the organism itself) are brought into contact with the antibody-peptide complex, the antigens displace the peptides, which can be detected by a change in fluorescence. In another aspect of the invention, suitable synthetic peptides are provided, as described below:
Overlapping 10x9-mer peptides were synthesis, mapping the p3 18-mer (i.e. 1-9aa, 2- 10aa, 3-11aa etc. of SEQ ID 11) to identify a target antigen to be utilised for the generation of the analogue library.

The sequences of the 10x9-mer peptides are shown below, using standard 1 letter amino acid coding:
SEQ ID 1: GRDIKVQFQ (1-9aa), denoted P782-1
SEQ ID 2: RDIKVQFQS (2-10aa), denoted P782-2
SEQ ID 3: DIKVQFQSG (3-1 laa), denoted P782-3
SEQ ID 4: IKVQFQSGG (4-12aa), denoted P782-4
SEQ ID 5: KVQFQSGGN (5-13aha), denoted P782-5
SEQ ID 6: VQFQSGGNN (6-14aa), denoted P782-6
SEQ ID 7: QFQSGGNNS (7-15aa), denoted P782-7
SEQ ID 8: FQSGGNNSP (8-16aa), denoted P782-8
SEQ ID 9: QSGGNNSPA (9-17aa), denoted P782-9
SEQ ID 10: SGGNNSPAV (10-18aa), denoted P782-10

These peptides, along with the p3 18-mer (SEQ ID 11) were screened against antisera (from rabbit) to the p3 18-mer (SEQ ID 11). The screen utilised the competitive ELISA (Enzyme-linked immunosorbant assay) technique, which was used to assess the relative binding affinities of the 9-mer peptides to the 18-mer peptide.

Briefly, the assay involved binding (coating) the p3 18-mer (P779, SEQ ID 11) to ELISA plates, followed by incubation with variables amounts of competing ligand (9-mers, SEQ ID 1-10) with the antisera (1:250 dilution). The level of binding of the antisera to p3 18- mer (P779, SEQ ID 11) was indicated by treatment with goat anti-rabbit peroxidase conjugated antibody and then by addition of a chromogenic substrate ABTS (2,2-azino- di-[3-ethylbenzthiazoline sulphonate]) and hydrogen peroxide where upon a green colour developed which was recorded as an optical density (absorbance) on a plate reader. The colour development is proportional to the level of binding of the primary antisera bound to the plate.

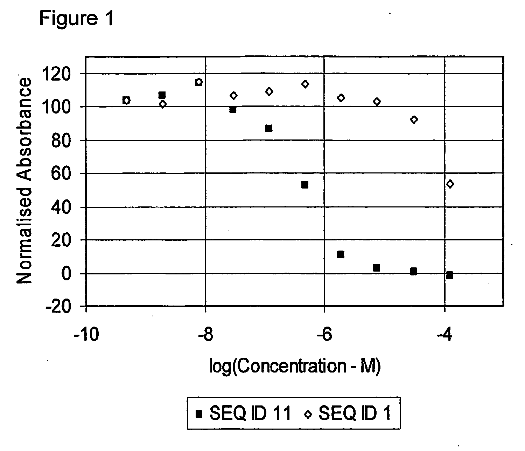

A test ELISA was performed in order to ascertain the concentrations of coating peptide and competing peptides for the Elisa experiments, comprising a competitive ELISA of 9-mer P782-1 (GRDIKVQFQ) - SEQ ID 1 - against 18-mer P779 (GRDIKVQFQSGGNNSPAV) - SEQ ID 11 (Mustafa, A.S. *et al, supra*)*.* This 9-mer was chosen as it also overlaps with the p2 18-mer (YLQVPSPSMGRDIKVQFQ) - SEQ ID 12. From the results, the inventors showed the concentrations of peptides used were suitable for the Elisa studies. ELISA plates were coated with 2 µM solution of p3 18-mer (P779) - SEQ ID 11 - and used 500 µM solutions of competing peptides, which were then subjected to four-fold serial dilution. Results from the test ELIZA are shown in Figure 1.

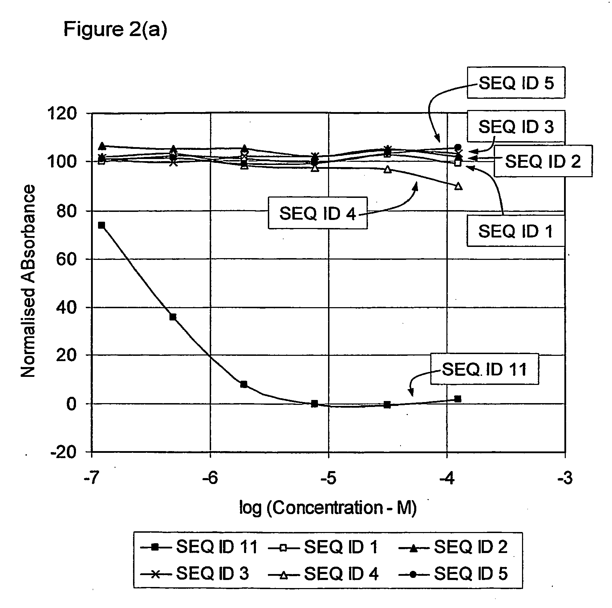

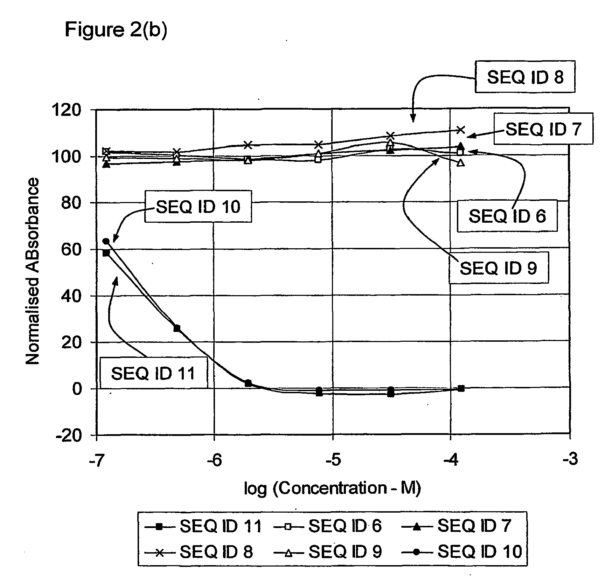

An Epitope Mapping ELISA was then performed, to determine relative binding affinities between the 10x9-mers (P782-1 to P782-10) - SEQ ID 1 to 10 - against the p3 18-mer (P779) - SEQ ID 11. The peptide concentration range selected (linear part of the competition curve) is important to get a qualitative assessment of the relative binding affinities of the competing epitopes. The results showed that 9-mers P782-1 to P782-9 (i.e. SEQ ID 1 to 9) did not appear to bind to the antisera to any significant extent in this concentration range; however the 9-mer P782-10 (SGGNNSPAV) - SEQ ID 10 - bound to the antisera as tightly as the p3 18-mer peptide (SEQ ID 11). This result is very significant and directed the inventors towards the possible importance of the C-terminal valine residue, as the preceding 9-mer P782-9 (QSGGNNSPA), SEQ ID 9, showed no significant binding even though it overlaps the P782-10 peptide, SEQ ID 10, by 8 residues. Results from this Epitope Mapping ELIZA are shown in Figures 2(a) and 2(b).

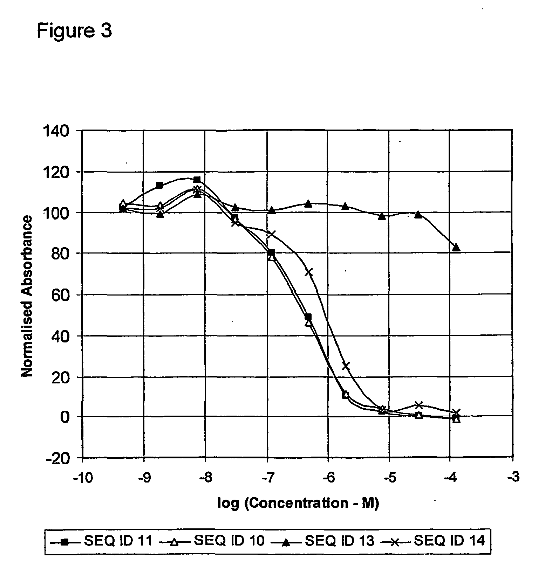

A Targeted Epitope ELISA was then designed to test the importance of the C-terminal region of the epitope on binding to the antisera. Two 6-mers were synthesied, P782-11 (GNNSPA) - SEQ ID 13 - and P782-12 (NNSPAV) - SEQ ID 14, which are truncated forms of their respective 9-mers and subjected these to competitive ELISA as before and also re-tested the 9-mer P782-10 (SEQ ID 10) identified as a significant epitope in the Epitope Mapping Elisa. Results from the Targeted Epitope ELIZA are shown in Figure 3. The data very clearly show reproducible results, and again indicated that:
- The 9-mer P782-10 (SEQ ID 10) bound to the antisera as tightly as the p3 18-mer peptide P779 (SEQ ID 11)
- The 6-mer P782-11 (SEQ ID 13) did not bind to the antisera and:
- The 6-mer P782-12 (NNSPAV) (SEQ ID 14) binds to the antisera 2-fold (1.99) less tightly relative to either 9-mer P782-10 (SEQ ID 10) or 18-mer P779 (SEQ ID 11).

This result shows the importance of the C-terminal region that contains the valine residue. On the basis of this experiment it would be unwise to modify the N-terminal region but instead to target the C-terminal valine residue in order to generate some differing binding affinities. The inventors therefore decided to focus the library based upon the 9-mer P782-10 (SGGNNSPAV), SEQ ID 10, and to modify the C-terminal region and synthesised peptides with the sequence SGGNNSPA-X where X is a variable amino acid including other hydrophobic and charged amino acids.

For the Library Elisa the inventors synthesised the following peptides:
SEQ ID 15: SGGNNSPAA, denoted P783-1
SEQ ID 16: SGGNNSPAF, denoted P783-2
SEQ ID 17: SGGNNSPAL, denoted P783-3
SEQ ID 18: SGGNNSPAM, denoted P783-4
SEQ ID 19: SGGNNSPAY, denoted P783-5
SEQ ID 20: SGGNNSPAG, denoted P783-6
SEQ ID 21: SGGNNSPAS, denoted P783-7
SEQ ID 22: SGGNNSPAP, denoted P783-8
SEQ ID 23: SGGNNSPAN, denoted P783-9
SEQ ID 24: SGGNNSPAE, denoted P783-10
SEQ ID 25: SGGNNSPAR, denoted P783-11

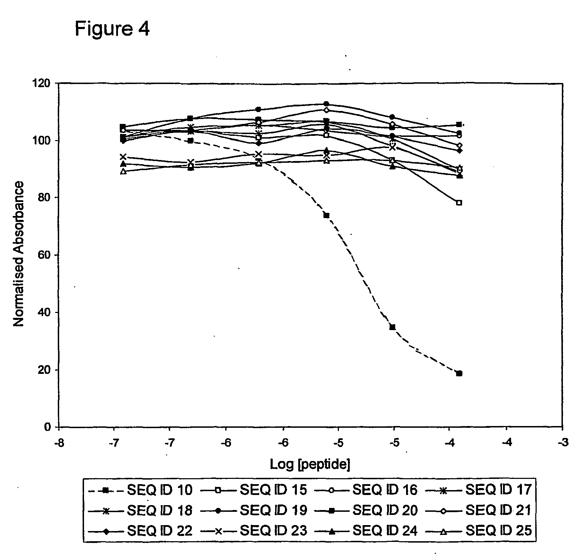

The inventors restricted the concentrations of the competing peptides within that utilised in the previous experiments, as it would not be possible to assess the relative binding affinities of the competing peptides within a single assay. The results from the competitive ELISA of the library (shown graphically in Figure 4) shows a range of binding affinities relative to the 9-mer P782-10 (SGGNNSPAV), SEQ ID 10. The epitopes of particular significance are the alanine, leucine and methionine variants (SEQ ID 15, 17 and 18) especially at epitope concentrations of 125 µM and 31.3 µM. Of these, the alanine and methionine variants are particularly preferred, as they have increased heat stability, making them particularly useful for field use in hot countries.

### ELISA Assay

A suitable method for carrying out an ELISA assay according to the present invention is as follows:
In order to determine the presence or absence of *Mycobacterium tuberculosis* a sample from a patient is contacted with an antibody raised against SEQ ID 11; the presence or absence of a complex formed between a peptide in the sample and said antibody is detected; and the amount of complex formed in said sample to a normal control, is detected, wherein an increase in the amount of complex in the sample compared to the control indicates the presence of *Mycobacterium tuberculosis.* General methods of performing ELIZA assays are known to the skilled addressee. Samples from patients may be provided in the form of body fluids such as blood, saliva, semen and milk or tissue samples such as biopsy material suitably prepared by e.g. homogenisation. However, samples having origin from the respiratory tract are preferred. Of particular preference are sputum, or fluids obtained by bronchial or pulmonary lavage. Most preferred, however, are samples obtained by inducing a cough reflex in a patient by nebulisation and collecting entrained material from a patient's breath; these samples contain material of tracheal origin.

### Displacement ELISA Assay

A suitable method for carrying out a displacement ELISA assay according to the present invention is as follows:
In order to determine the presence or absence of Mycobacterium tuberculosis, a composition containing a peptide as described herein, and defined according to the claims, and a sample from a patient is provided. The composition is contacted with an antibody raised against SEQ ID 11; the presence or absence of a complex formed between said peptide and said antibody is detected; and the amount of complex formed in said sample is compared to a normal control, wherein an decrease in the amount of complex in the sample compared to the control indicates the presence of *Mycobacterium tuberculosis.*

General methods of performing displacement ELIZA assays are known to the skilled addressee. Again, samples from patients may be provided in the form of body fluids such as blood, saliva, semen and milk or tissue samples such as biopsy material suitably prepared by e.g. homogenisation. However, samples having origin from the respiratory tract are preferred. Of particular preference are sputum, or fluids obtained by bronchial or pulmonary lavage. Most preferred, however, are samples obtained by inducing a cough reflex in a patient by nebulisation and collecting entrained material from a patient's breath; these samples contain material of tracheal origin.

## Claims

1. A peptide consisting of any of the following sequences:
(SEQ ID 15)
SGGNNSPAA;
(SEQ ID 17)
SGGNNSPAL;
(SEQ ID 18)
SGGNNSPAM.

2. A peptide according to claim 1, further comprising a label.

3. An antibody that specifically binds to SEQ ID 11, or a fragment of said antibody, wherein said fragment specifically binds to SEQ ID 11.

## Patentansprüche

1. Peptid bestehend aus einer der folgenden Sequenzen:
• (SEQ ID 15) SGGNNSPAA;
• (SEQ ID 17) SGGNNSPAL;
• (SEQ ID 18) SGGNNSPAM.

2. Peptid nach Anspruch 1, weiter umfassend ein Label.

3. Antikörper, der sich spezifisch an SEQ ID 11 bindet, oder ein Fragment des Antikörpers, wobei sich das Fragment spezifisch an SEQ ID 11 bindet.

## Revendications

1. Un peptide consistant en quelqu'une des séquences suivantes :
(SEQ ID 15) SGGNNSPAA ;
(SEQ ID 17) SGGNNSPAL ;
(SEQ ID 18) SGGNNSPAM.

2. Un peptide selon la revendication 1, comprenant en outre une étiquette.

3. Un anticorps qui se lie d'une manière spécifique au SEQ ID 11, ou un fragment dudit anticorps, dans lequel ledit fragment se lie d'une manière spécifique au SEQ ID 11
